# EUROPEAN PATENT APPLICATION

(11) **EP 2 327 431 A1**
(43) Date of publication of application: **01.06.2011**
(21) Application number: 09177081.8
(22) Date of filing: 25.11.2009
(51) Int. Cl.: A61M 5/145, A61M 5/168

(54) **Medical delivery device**

(71) Applicant: Letcat Aktiebolag, 223 69 Lund (SE)
(72) Inventor: Larsson, Roland, 245 44, STAFFANSTORP (SE); Aronsson, Torbjörn, 217 41, MALMÖ (SE); Johansson, Mathias, 224 79, LUND (SE)
(74) Representative: Bergström, Johan Erik

(57) **Abstract**

The present invention relates to a medicament delivery device comprising a housing (8); a medicament container (18) arranged inside said housing (8), a drive mechanism (12, 14, 44) comprising a plunger rod (12) capable of acting on a stopper (16) inside said medicament container (18) for expelling a dose of medicament, said drive mechanism further comprising a force member (14) acting on said plunger rod (12), an actuation mechanism (10) capable of releasing said drive mechanism upon activation, dose setting means (42) capable of limiting the dose expelled from the medicament container (18), - electronics (51) capable of handling information signals. The invention is characterised in that it further comprises a first sensor unit (49) connected to said electronics (51), that said electronics (51) is provided with information regarding drug volume of said medicament container, that said first sensor unit (49) is capable of sensing an initial start position and sensing and measuring the actual position of the plunger rod (12) and that said first sensor unit (49) is capable of transmitting said positions to said electronics (51), whereby said electronics (51) is capable of calculating remaining dose quantity in the medicament container (18).

## Description

### TECHNICAL AREA

The present invention relates to a medical delivery device and in particular an injector for self-administration of medicament.

### TECHNICAL BACKGROUND

Medicament delivery devices specially designed and intended primarily for self-administration have been developed during a number of years and numerous different solutions have been invented for this purpose.

In order that the patient or user is alerted regarding the state of the device for example regarding the number of doses that have been delivered or are remaining as well as the dose size, if the user may set different dose sizes, some devices are arranged with indicia visible through openings or windows in the device for mechanical dose information mechanisms or electronic displays if the device is provided with electronic dose information mechanisms.

Regarding mechanical dose information mechanisms, indicia are often arranged on an outer surface of a rotatingly arranged member, which during dose setting and/or dose delivery rotates a certain amount, which is displayed in the opening or window of the device. This function is adequate for many devices where the doses are rather large and/or that the rotation of the dose information member is to such an extent that all the indicia to be shown can fit onto the surface of the member.

However, for some devices and in particular when the dose increments are rather small, or the movement of the indicia member is rather small for a set dose, all indicia to be shown cannot fit onto the surface of the member which leads to problems regarding providing the user with the appropriate information. This is also true for many electro-mechanical solutions using e.g. protrusions and valleys acting on breakers or contacts, i.e. opening and closing of contacts in order to create unique combinations. The mechanics of this type of contacts or switches cannot be made very small in order to cope with small dose increments.

In order to handle small dose increments such that the proper information is provided to the user some pure electronic contact-less solutions have been provided.

W02008037801 discloses an injection device having a dose setting mechanism comprising linear capacitive sensors. The measurement is performed by analogue capacitance measurements which are then converted to digital form. The means for electronically detecting the amount of a set dose and/or the means for electronically detecting the amount of an injected dose may comprise at least two substantially disc shaped members being arranged with a substantially fixed mutual distance along a longitudinal direction of the injection device, said substantially disc shaped members being rotationally movable relatively to each other during dose setting and/or injection, and an angular displacement between said substantially disc shaped members may in this case be indicative of the amount of a set dose and/or the amount of an injected dose.

W02007141225 discloses a medication delivery device having a dose setting mechanism which comprises first and second transmitter electrodes and a receiver electrode positioned on the inner surface of a housing and an axially movably member having a reflector electrode, wherein the reflector electrode is adapted to be capacitively coupled to at least one of the transmitter electrodes and to the receiver electrode, such that when said transmitter electrodes are provided with transmitter signals the axial position of the movable member is detected by the receiver electrode. The measurement is performed by analogue capacitance measurements which are then converted to digital form.

However, none of the disclosed documents provide the possibility of knowing the exact position of the plunger rod in relation to the device in order to be able to exactly keep track of remaining volume of medicament in the cartridge. Neither do the disclosed devices have a function indicating that an injection has been paused nor are they able of indicating that there is no cartridge in the device at all.

### BRIEF DESCRIPTION OF THE INVENTION

The aim of the present invention is to provide a medicament delivery device that both handles the drawbacks with the state of the art solutions and as well provides an accurate function regarding the position of the plunger rod.

This aim is obtained by the features of the independent patent claims. Preferable embodiments of the present invention are found in the dependent patent claims.

According to a main aspect of the invention it is characterised by a medicament delivery device comprising a housing; a medicament container arranged inside said housing, a drive mechanism comprising a plunger rod capable of acting on a stopper inside said medicament container for expelling a dose of medicament, said drive mechanism further comprising a force member acting on said plunger rod, an actuation mechanism capable of releasing said drive mechanism upon activation, dose setting means capable of limiting the dose expelled from the medicament container, electronics capable of handling information signals, characterised in that it further comprises a first sensor unit connected to said electronics, that said electronics is provided with information regarding drug volume of said medicament container, that said first sensor unit is capable of sensing an initial start position and sensing and measuring the actual position of the plunger rod and that said first sensor unit is capable of transmitting said positions to said electronics, whereby said electronics is capable of calculating remaining dose quantity in the medicament container.

According to another aspect of the invention, it further comprises a second sensor unit capable of sensing and measuring the dose set with the dose setting means.

According to a further aspect of the invention, said dose setting means performs a linear movement during the setting of said dose and that said second sensor unit is capable and arranged to measure linear movements.

According to yet an aspect of the invention, said first and/or second sensor unit comprises at least one printed circuit board, having means to collect information from said first and/or second sensor unit.

According to yet a further aspect of the invention, said at least one printed circuit board comprises a display, capable of providing a user of the device with information.

According to another aspect of the invention, said first and/or said second sensor units comprise any or combinations of capacitive, resistive, optical, inductive, magnetic sensor technologies.

The present invention has a number of advantages in comparison with the known devices in this technical area. The first sensor unit provides the possibility of determining the exact position of the plunger rod in relation to the initial reference position. This together with provided information regarding the size of the medicament container it is possible to sense, measure and calculate the drug volume remaining in the medicament container. Thus the device measures the distance from the initial reference position to the position when the plunger rod is moved in contact with the stopper of the medicament container, from which position the drug volume may be calculated based on the distance the plunger rod is moved during each dose delivery in that the actual position of the plunger rod is always known.

This provides the advantage to have control over the injection sequence and to provide the user, and possibly other medical staff, with accurate dose delivery information.

When the second sensor unit is utilized for devices having adjustable dose sizes, the second sensor unit provides the possibility of obtaining information regarding the dose size to be delivered, i.e. the set dose. Because this information is available to the electronics as well as information from the first sensor unit measuring the movement of the plunger rod, it is possible to obtain information regarding if somehow the full dose was not delivered because it is possible to compare the distance information from both units, i.e. the distance of the set dose when this information is compared with the distance the plunger rod actually has travelled during delivery. Since the electronics have information from both sensor units, and both sensor units measure the distance the plunger rod moves during dose delivery, on the one hand the electronics may choose from which sensor unit information shall be obtained and on the other hand this provides a higher degree of safety or reliability since the both values are compared.

Preferably the movements of the components that are measured with the first and/or second sensor units move linearly which is an advantage in terms of positioning sensors and sensing indicia or indications. Due to this it is possible to use planar printed circuit boards on which the sensor components are placed, which provides a simple and yet cost effective solution. Preferably also a display means is connected more or less directly to the circuit board, which display means is capable of providing information to the user.

The sensor units could utilize many different and per se known technologies where some have certain advantages and disadvantages and where the choice of technology may depend on the application and desired function, such as accuracy, cost-efficiency, power consumption, reliability, just to mention a few. In all a new and inventive handling of dose delivery information of a medicament delivery device is obtained with the present invention.

These and other aspects of and advantages with the present invention will become apparent from the following detailed description and the accompanying drawings.

### BRIEF DESCRIPTION OF THE DRAWINGS

In the detailed description, reference will be made to the accompanying drawings, of which
Fig. 1 is a schematic view of some important functions of a medicament delivery device,
Figs. 2 and 3 schematically show positions of sensor units according to the present invention,
Figs. 4-5 show schematically one type of sensor system comprised in the present invention,
Figs. 6-8 show another type of sensor system according to the invention,
Fig. 9 shows a further type of sensor system according to the invention,
Fig. 10 shows yet a type of sensor system according to the invention, and
Fig. 11 shows yet another type of sensor system according to the invention.

### DETAILED DESCRIPTION OF THE INVENTION

In the present application, when the term "distal part/end" is used, this refers to the part/end of the delivery device, or the parts/ends of the members thereof, which under use of the delivery device, are located the furthest away from the medicament delivery site of the patient. Correspondingly, when the term "proximal part/end" is used, this refers to the part/end of the delivery device, or the parts/ends of the members thereof, which under use of the delivery device, are located closest to the medicament delivery site of the patient.

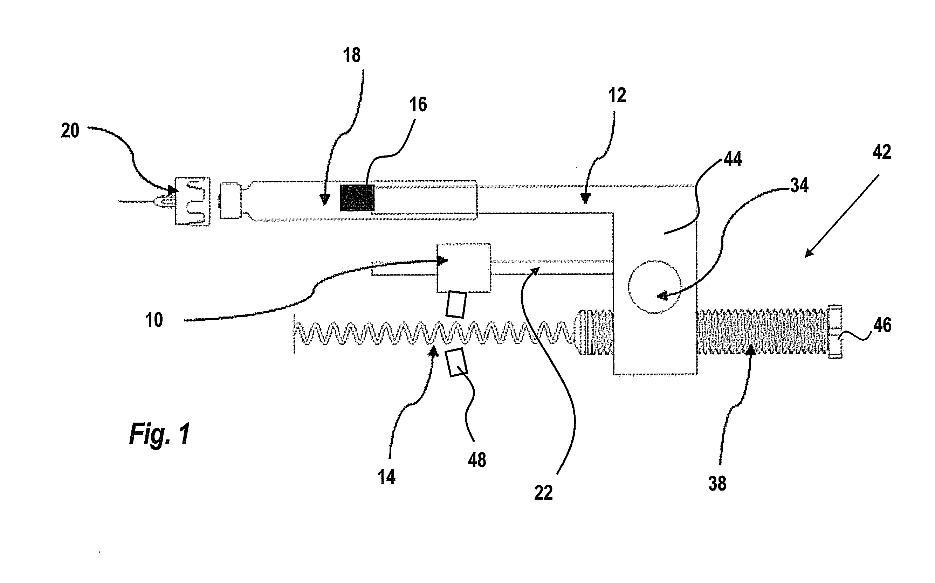

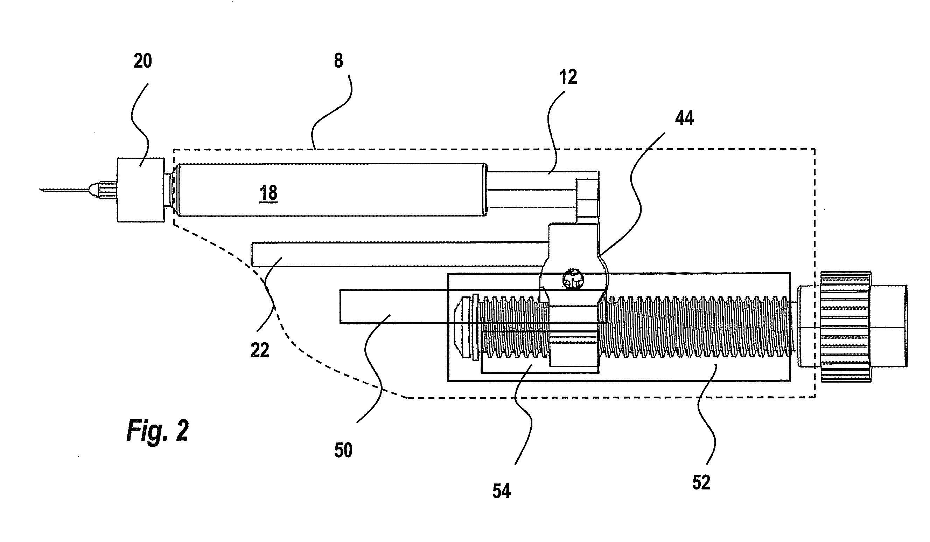

Figure 1 shows a schematic view of a non-limiting example of a medicament delivery device that could utilize the present invention. Components not important for the understanding of the present invention are removed for clarity, such as an activation button, a needle shield etc. Also, the housing 8, Fig. 2, of the device is shown with broken lines for clarity. The device shown comprises a dose release brake 10. It is designed as a locking unit that is capable of locking and releasing a plunger rod 12, which plunger rod 12 is urged in the proximal direction by a loaded medicament delivery force means 14, such as e.g. a spring, a gas cylinder or the like. The plunger rod 12 is urged against a movable stopper 16 arranged inside a medicament container 18. A medicament delivery member 20 may be attached to the medicament container 18, such as an injection needle as shown. It is however to be understood that other types of medicament delivery members may be utilized such as mouth or nose nozzles, aerosol creating membranes, nebulizers or the like.

The dose release brake 10 could be designed such that it may be locked and released in any position from an initial position to an end position. The locking unit may be acting either directly on the plunger rod 12 or on a separate rod 22, hereafter named dose release brake rod, fixed in relation to the plunger rod and arranged generally parallel with the plunger rod.

The general function is that the dose release brake 10 will keep the dose release brake rod 22 and thus the plunger rod 12 fixed while the user is setting a dose, as will be described. When a medicament delivery is performed the dose release brake 10 is opened by an appropriate release mechanism such as an injection button and the set dose of the drug is delivered from the medicament container 18. According to one example of the dose release brake 10, it should allow movement in one direction only.

Another feature of the device is a cartridge exchange brake 34. The function of the cartridge exchange brake is to allow the plunger rod 12 to be moved in the distal direction to an initial reference position, when a used medicament container 18 is to be replaced by a new container, such that the plunger rod is disconnected from the medicament delivery force unit 14 during the exchange. After the exchange, the plunger rod 12 is moved in the proximal direction until it is moved in contact with the stopper 16 of the medicament container and the cartridge exchange brake 34 again locks the plunger rod operationally to the medicament delivery force unit 14.

The medicament delivery device shown further preferably comprises a dose setting unit 42, which is operationally connected to the plunger rod 12 via a transversal support member 44, hereafter named plunger rod housing, to which the plunger rod 12 and the dose release brake rod 22 are attached. The dose setting unit comprises a threaded dose setting screw 38 that is threadidly engaged with the plunger rod housing 44. The distal end of the dose setting screw 38 is arranged with a dose setting knob 46, which may be operated manually by a user such that the dose setting screw is moved in the distal direction, whereby the proximal end of the dose setting knob is moved out of contact with a fixed stop surface 48. The distance between the proximal end of the dose setting screw 38 and the stop surface 48 corresponds to a certain dose quantity of medicament.

According to the present invention, in order to provide the user with dose delivery information, a sensor system could be included in a medicament delivery device as described above.

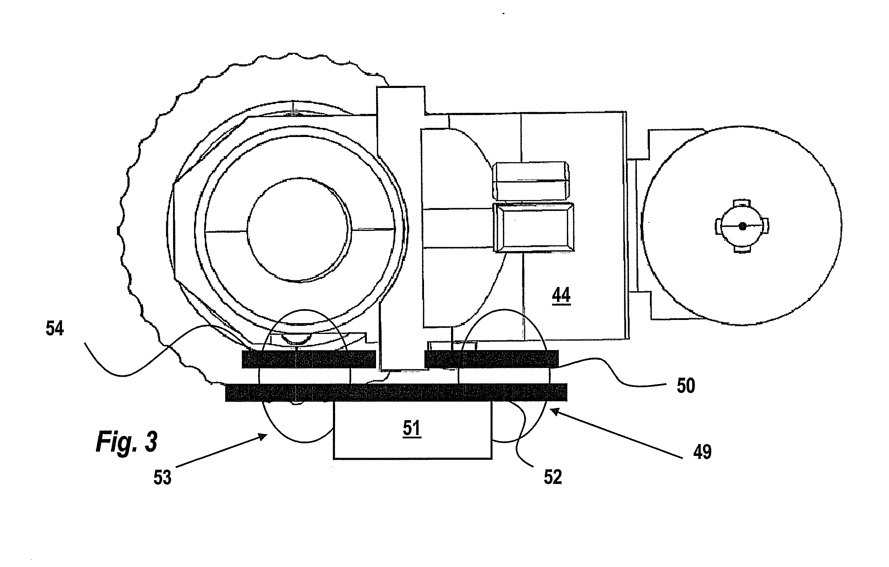

The sensor system could comprise a first sensor unit 49 comprising a first sensor scale 50, Figs. 2 and 3, hereafter named plunger rod scale attached to the plunger rod housing 44 or any other component that moves when the plunger rod 12 moves. It is positioned adjacent a printed circuit board 52 attached to the housing of the device or any other fixed part of the device, where a first sensor member is positioned, capable of sensing the plunger rod scale 50. The circuit board is further provided with electronics 51 capable of handling information retrieved from sensors, information obtained from input means as well as information stored in memories.

The electronics 51 could comprise, on the printed circuit board, either in one piece or divided into several pieces depending on space requirements, all necessary circuits, processors, memory storage means, I/O functions, display means, buttons, and possibly wired or wireless communication means for performing all the desired functions that may be requested by different users. In this aspect there is of course some sort of power source that is capable of operating the electronics 51 of the device, which may comprise batteries as well as photo-voltaic panels. Further, the communication means may comprise means that utilize wired and/or wireless telephone networks, WLAN, etc., i.e. any suitable communication means, whereby it is possible to transfer information from the device to for instance a computer of a physician for monitoring the patient's use of the device.

The first sensor unit 49 is intended to measure and provide information regarding the actual position of the plunger rod from an initial position where it is in its most distal position, to its most proximal position where the medicament container has been emptied. That is, the first sensor unit 49 is capable of sensing the actual position of the plunger rod in relation to the device where the main purpose is to determine the remaining dose quantity of the medicament container. Preferably the first sensor unit is capable of measuring a distance further than the position when a medicament container has been emptied. This then gives the possibility of obtaining information that there is no container present in the device.

The electronics 51 is thus capable of determining the actual drug volume of the medicament container by information from the first sensor unit and thus the remaining drug volume. The first sensor unit will thus provide information to the electronics 51 of the distance from the initial reference position to the position when the proximal part of the plunger rod is moved in contact with the stopper of the medicament container. Since the electronics 51 have been provided with data regarding the size of the medicament container that could be stored in a memory of the electronics, the electronics 51 has all information needed to calculate the remaining drug volume of the medicament container.

The sensor system could also comprise a second sensor unit 53 comprising a second sensor scale 54 arranged to the dose setting screw and positioned adjacent the printed circuit board 52 where a second sensor member is positioned. The second sensor unit is intended to measure and indicate the distance the dose setting screw is moved during setting of the desired dose, i.e. how far the dose setting screw 38 is moved in the distal direction during turning of the dose setting knob 46, i.e. during setting of dose. This information is available to the electronics 51 such that when the dose is delivered the first sensor unit measures the distance it has moved and this information is compared by the electronics 51 with the set dose value provided from the second senor unit. A miss-match indicates that a set and desired dose has not been delivered. The electronics 51 could then alert the user regarding this via the display and/or by other means such as audible alerts. Thus it is possible to obtain information both regarding the size of the current dose to be injected and the amount of remaining dose volume in the medicament container. By the above it is of course possible for the electronics 51 to use data from either of the sensor units or both, when the medicament delivery movement is performed by the plunger rod.

There are a number of different technologies to be used, where the main advantage with the solution according to the present invention is that there is a linear movement of the interacting components, which facilitates the setup of sensors and measuring functions.

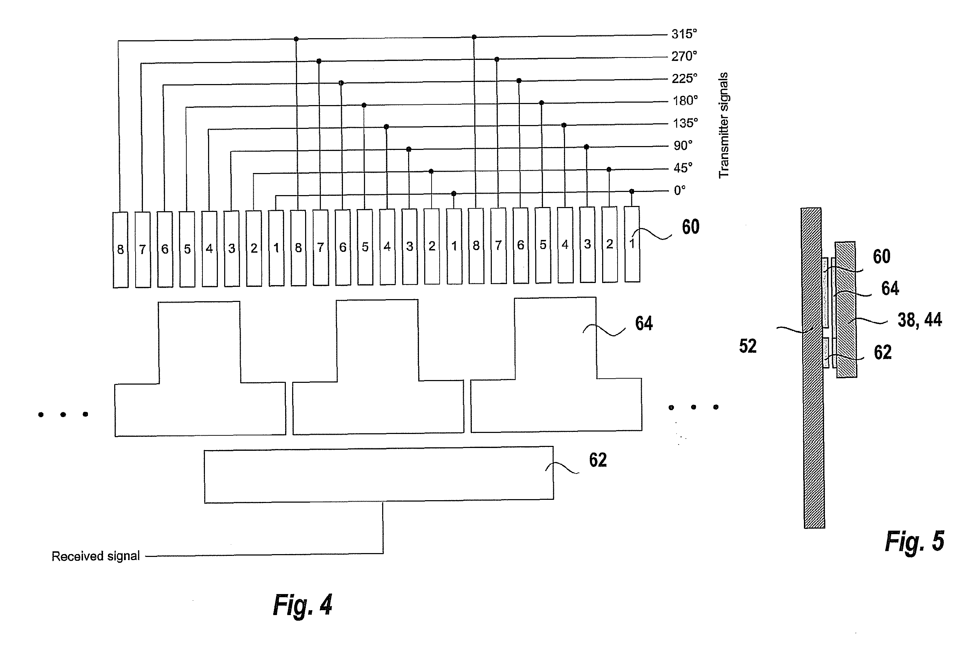

One example of technology that can bee used is capacitive sensor systems. As seen in Figs. 4 and 5, the capacitive sensor uses a plurality of transmitter plates 60 capacitively coupled to one or more pickup plates 62 via a series of conductive plates on the moving part, the transferring plates 64. The capacitance in this arrangement becomes quite small and in order to amplify the received signal, several groups of transmitter plates 60 may be placed along the travelling path of the moving part 38, 44.

The transmitter plates 60 are supplied with signals, each phase shifted relative to the other. These signals can be sine shaped, digital pulses of different duration that roughly represent sine waves, or any other suitable waveform.

The transferring plates 64, located on a moving part such as the plunger rod housing 44 or the dose setting screw 38, comprise a series of conductive patterns. A part of the transferring pattern is located directly over some of the transmitter plates 60 and pick up the signals sent to the transmitter plates 60. Other parts of the transferring plate 64 are located over the pickup plates 62 that pick up the signals transmitted from the transmitting plates 60. The signal received at the pick up plate 62 is used to determine the movement and position of the moving part 38, 44.

It is obvious that the design can be made the other way around with the transmitting plates 60 and the pick up plates 62 on the moving part 38, 44 and the transferring plates 64 on the circuit board 52.

In the present design the following configuration is used. Several groups of eight transmitter plates 60 are used. These transmitting plates have a rectangular shape like the ones shown in Figure 4. The signals sent to the transmitting plates are digital pulses of altering durations that represent sine waves. The signals are phase shifted 45° in relation to each other.

As seen in Fig. 4, the transferring plates 64, located on the moving part 38, 44, is a series of "T" shaped conductive patterns. The narrower part of the transferring pattern is located directly over the transmitting plates 60 and picks up four of the eight signals and mixes them together to form a sum of the signals applied to the transmitter plates 60. The wider part of the transferring pattern is located directly above the pickup plate 62. The pickup plate 62 is a rectangular conductive plate. Note that Figure 4 shows the different plates in a flattened view to make it easier to understand, Figure 5 shows the correct arrangement in a side view.

The signal received on the pickup plate 62 is the sum of four of the eight sine shaped waveforms applied to the transmitting plates 60. Depending on the location of the transferring plates 64 relative to the transmitting plates 60, different signals are transferred from the transmitting plates 60 to the pick up plate 62. After amplification and sampling, a sine wave, phase shifted relative to a known reference signal, is obtained. By measuring this phase shift, the position of the scale in relation to the transmitter plates may be calculated. The transmitter plates 60 and the pickup plate 62 are preferably realized as copper pattern on the same circuit board 52. The transferring plates are located on the plunger rod housing which position is to be determined. As mentioned above, in the device according to the present invention, two sets of sensors are used.

As the moving scale moves along the transmitting 60 and pickup plates 62, the phase shift will go from 0° to 360°. If the moving scale continues to move in the same direction, the phase shift will start over from 0° again. The electronics 51 will keep track of how many periods that have passed.

The capacitive sensor has the advantage that its power consumption can be kept very low.

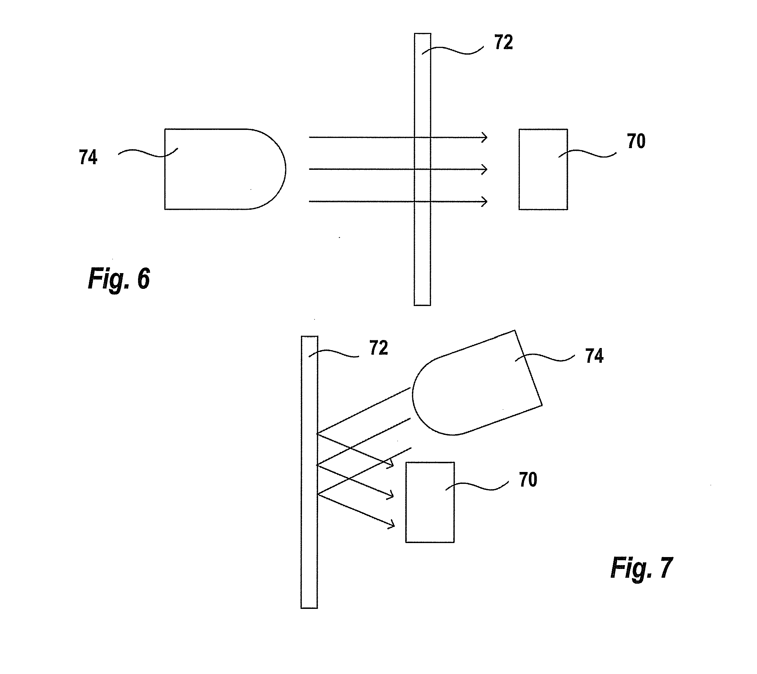

Another example of determining the position of the moving parts in relation to a fixed part of the injector, preferably the main circuit board 52, is to use an optical linear sensor array 70, Figs. 6 and 7. This sensor array is like a camera sensor but with only one line of pixels. The sensor is located on the main circuit board 52 and a scale pattern 72 is located on the moving part 38, 44. The scale pattern 72 can be designed in a way that makes it possible to obtain a unique code for each possible position of the moving part. The pattern can also be designed for relative measurements.

The scale has to be illuminated by a light source 74. Both transmitted light Fig. 6 and reflected light Fig. 7 can be used using either a transmissive scale pattern or a reflective one. In the reflective case, the code on the scale is built up using a pattern of reflective and non reflective areas e.g. black and white areas. A transmissive scale is built up using a pattern of transmissive and opaque areas.

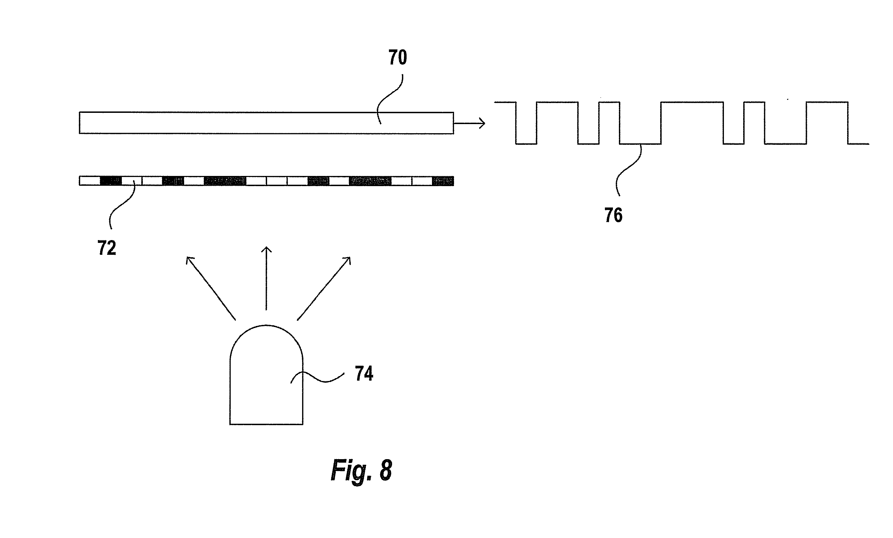

The signal obtained from the sensor, reference numeral 76 in Fig. 8 is a one dimensional image of the code on the scale. Of course, an ordinary, two dimensional image sensor array can also be used for the same purpose but that will probably be more expensive, take up more space and be more power consuming.

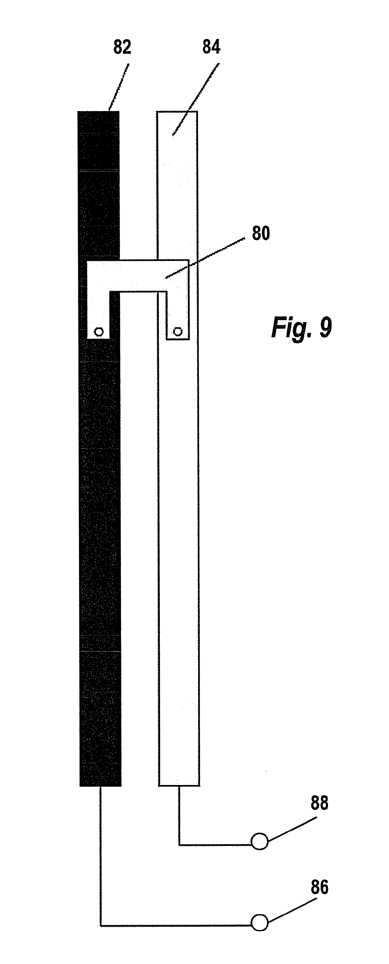

A further possible way to detect the relative position of one part of the injector in relation to another is shown in Fig. 9. Here a resistive position sensor is used. The principle of this sensor is the same as a potentiometer, a wiper contact 80 slides along a resistive path 82 and the resistance between the wiper contact and one end of the resistive path is measured. The measured resistance indicates the position.

In the case with linearly moving parts, two paths 82, 84 may be printed directly on the main circuit board, one resistive path 82 and one conductive path 84. The part moving in relation to the main circuit board is equipped with the wiper contact 80. This wiper contact connects the conductive path 84 with the resistive path 82. When the moving part moves, the resistance measured between the end point 88 of the conductive path 84 and the end point 86 of the resistive path 82 will increase or decrease depending on the direction the moving part moves.

By using two paths on the main circuit board 52, one resistive and one conductive, it is not necessary to connect to the wiper contact 80 itself and this is a major advantage since it simplifies the assembly of the device. If the conductive path 84 is omitted, the wiper contact 80 has to be connected to the main circuit board using some flexible connection like a thin wire or an FPC (Flexible Printed Circuit) and this will make the assembly of the device more complicated and thus more expensive.

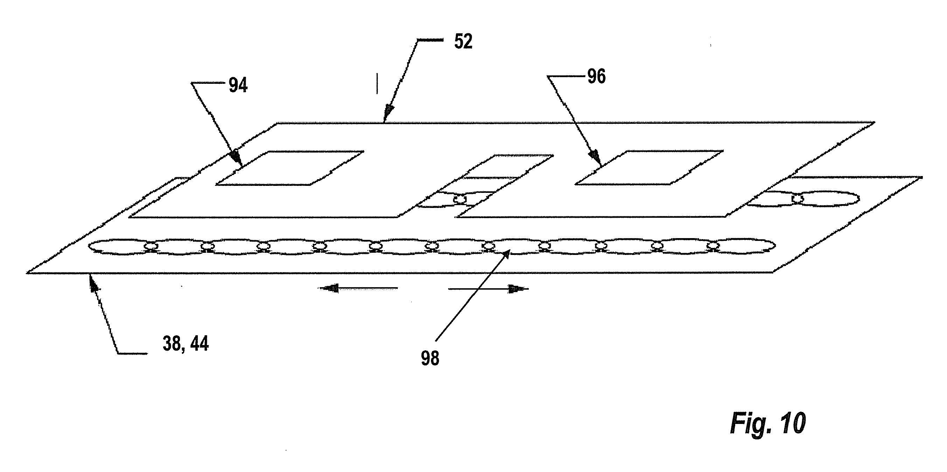

Another technology of measuring the relative position between parts is shown in Fig. 10 where induction is used. The unit used to measure movement between the moving part 38, 44 and the printed circuit board 52 in Fig. 10 comprises of one or more transmitting coils 94 and one or more receiving coils 96 inductively coupled to a set of single or plural passive coils 98. The passive coil(s) 98 on the circuit board 52 are oriented/placed in such way that when the emitting coil(s) 94 on the circuit board 52 are fed with an appropriate signal a phase shift that is proportional to the relative movement between the transmitting 94 and receiving coils 96 can be observed. The phase shift is then the measure of the relative movement between the transmitting/receiving coils 94, 96 and the passive ones 98.

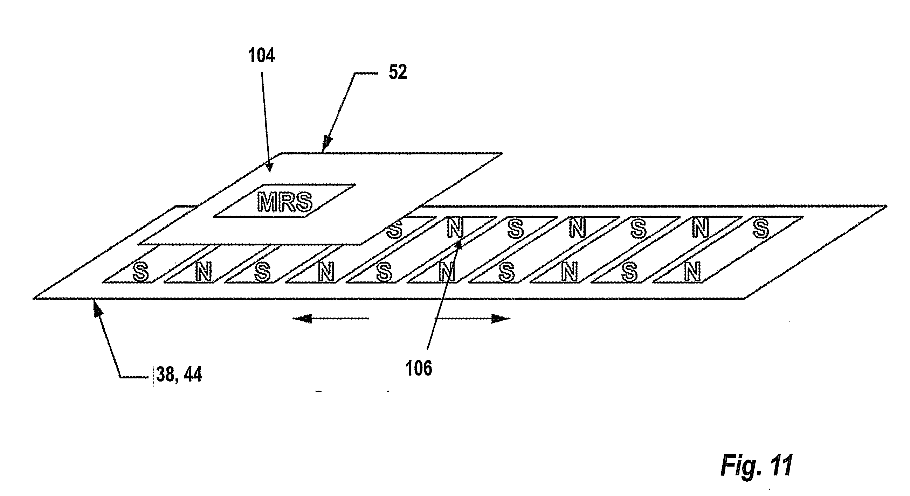

Yet a technology of measuring the relative position between parts is shown in Fig. 11 comprising a magnetic solution. The unit used to measure movement between the moving part 38, 44 and the circuit board 52 in Fig. 11 comprises one or more Magneto Resistive Sensor(s) (MRS) 104 on the circuit board and a surface with a magnetic pattern 106 on the moving part 38, 44. The MRS 104, single or plural, senses the magnetic pattern 106 in the surface, and is hereby able to measure the movement between the MRS and the said surface. The measurement of movement could be either relative or absolute depending on the pattern used, and the type of MRS. It is understood that a Hall-effect sensor also could be used instead of the MRS.

It is to be understood that for the above mentioned solutions, the components could change place such that the active components are placed on the moving part and the passive components are placed on the circuit board. However, this then requires some flexible wiring between the electronics and the active components.

It is to be understood that the embodiments of the invention described above and shown in the drawings are to be regarded only as non-limiting examples of the invention and that it may be modified in many ways within the scope of the patent claims.

## Claims

1. Medicament delivery device comprising
- a housing (8);
- a medicament container (18) arranged inside said housing (8),
- a drive mechanism (12, 14, 44) comprising a plunger rod (12) capable of acting on a stopper (16) inside said medicament container (18) for expelling a dose of medicament,
- said drive mechanism further comprising a force member (14) acting on said plunger rod (12),
- an actuation mechanism (10) capable of releasing said drive mechanism upon activation,
- dose setting means (42) capable of limiting the dose expelled from the medicament container (18),
- electronics (51) capable of handling information signals,
**characterised in that** it further comprises a first sensor unit (49) connected to said electronics (51),
- that said electronics (51) is provided with information regarding drug volume of said medicament container,
- that said first sensor unit (49) is capable of sensing an initial start position and sensing and measuring the actual position of the plunger rod (12) and
- that said first sensor unit (49) is capable of transmitting said positions to said electronics (51), whereby said electronics (51) is capable of calculating remaining dose quantity in the medicament container (18).

2. Medicament delivery device according to claim 1, further comprising a second sensor unit (53) capable of sensing and measuring the dose set with the dose setting means (42).

3. Medicament delivery device according to claim 1 or 2, wherein the first sensor unit is capable of measuring a distance further than the position when a medicament container has been emptied, thereby providing information that there is no container present in the device.

4. Medicament delivery device according to claim 2, wherein the electronics is capable of comparing the dose set from the second sensor unit with the distance measured by the first and/or second sensor unit during delivery of a dose, wherein a miss-match indicates an incomplete dose delivery.

5. Medicament delivery device according to claim 2, wherein said dose setting means (42) performs a linear movement during the setting of said dose and that said second sensor unit (53) is capable and arranged to measure linear movements.

6. Medicament delivery device according to claim 2, wherein said first and/or second sensor unit comprises at least one printed circuit board (52), having means to collect information from said first and/or second sensor unit (49, 53).

7. Medicament delivery device according to claim 6, wherein said at least one printed circuit board (52) comprises a display, capable of providing a user of the device with information.

8. Medicament delivery device according to claim 1, wherein said first and/or said second sensor (49, 53) units comprise any or combinations of capacitive, resistive, optical, inductive, magnetic sensor technologies.
